(19) Europäisches Patentamt
European Patent Office
Office européen des brevets

(11) EP 1 541 127 B1

(12) FASCICULE DE BREVET EUROPEEN

(45) Date de publication et mention de la délivrance du brevet:
**31.12.2008 Bulletin 2009/01**

(21) Numéro de dépôt: **04292906.7**

(22) Date de dépôt: **07.12.2004**

(51) Int Cl.:
***A61K 8/97*** *(2006.01)* ***A61K 8/11*** *(2006.01)*
***A61K 8/55*** *(2006.01)* ***A61K 8/67*** *(2006.01)*
***A61K 8/31*** *(2006.01)* ***A61K 8/60*** *(2006.01)*
***A61Q 19/00*** *(2006.01)*

(54) **Utilisation des modulateurs d'aquaglycéroporines comme amincissant**

Verwendung von Aquaglyceroporin-Modulatoren als Schlankheitsagentien

Use of modulators of aquaglyceroporins as slimming agents

(84) Etats contractants désignés:
**AT BE BG CH CY CZ DE DK EE ES FI FR GB GR HU IE IS IT LI LT LU MC NL PL PT RO SE SI SK TR**

(30) Priorité: **10.12.2003 FR 0351015**

(43) Date de publication de la demande:
**15.06.2005 Bulletin 2005/24**

(73) Titulaire: **L'ORÉAL**
**75008 Paris (FR)**

(72) Inventeur: **Cals-Grierson, Marie-Madeleine**
**92190 Meudon (FR)**

(74) Mandataire: **Allab, Myriam**
**L'OREAL - D.I.P.I.**
**25-29 Quai Aulagnier**
**92600 Asnières (FR)**

(56) Documents cités:
**WO-A-00/30603**
**WO-A-01/37799**
**WO-A-01/64177**
**WO-A-92/15293**
**WO-A-02/089758**
**FR-A- 2 578 165**
**FR-A- 2 774 589**
**FR-A- 2 790 645**
**FR-A- 2 819 409**
**US-A- 5 194 259**
**US-A1- 2002 106 388**
**US-B1- 6 399 089**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

Printed by Jouve, 75001 PARIS (FR)

## Description

La présente invention concerne l'utilisation de modulateurs d'aquaglycéroporines comme agent amincissant, et des compositions cosmétiques contenant de tels modulateurs.

Les rondeurs et/ou surcharges pondérales sont liées au dysfonctionnement de certaines cellules de l'hypoderme, appelées adipocytes, qui contiennent des quantités variables de graisses stockées sous la forme de triglycérides. Ces triglycérides sont synthétisés *in vivo* par les adipocytes eux-mêmes, selon des réactions de type enzymatique (lipogénèse), à partir des acides gras libres contenus dans le sang sous forme de lipoprotéines et du glucose apporté notamment par l'intermédiaire de certains aliments. La libération des acides gras à partir des lipoprotéines se fait à l'aide d'une enzyme, la lipoprotéine lipase, contenue dans les adipocytes ou via des récepteurs cellulaires aux lipoprotéines HDL, LDL ou VLDL. La transformation du glucose conduit soit à la formation de glycérol, soit à la formation d'acides gras libres par l'intermédiaire d'une enzyme spécifique, l'acétylCoA-carboxylase qui transforme le glucose en acétyl-CoA puis en acides gras.

Or, parallèlement, les triglycérides ainsi formés, puis stockés, dans les cellules des adipocytes peuvent également se dégrader (lipolyse), toujours sous l'action d'enzymes spécifiques, les triglycérides lipases, contenues dans ces mêmes cellules, et qui sont susceptibles d'être activées par l'AMP cyclique. L'AMP cyclique est régulé par l'adényl cyclase et susceptible d'être hydrolysé en 5'AMP par la phosphodiestérase. Ce mécanisme de lipolyse conduit à la libération d'acides gras d'une part et de glycérol et/ou de mono-et/ou di-esters du glycérol d'autre part.

Les acides gras ainsi relargués peuvent alors soit diffuser dans l'organisme pour y être consommés ou transformés de différentes façons, soit être re-captés (aussitôt ou un peu plus tard) par les adipocytes pour générer à nouveau des triglycérides par lipogénèse.

Si, pour des raisons diverses (nourriture trop riche ou déséquilibrée, sédentarité, variation du métabolisme, vieillissement et autres), un déséquilibre substantiel s'installe dans l'organisme entre la lipogénèse (formation de triglycérides par réaction enzymatique entre des acides gras et le glycérol) et la lipolyse (décomposition enzymatique de triglycérides en acides gras et glycérol), c'est-à-dire plus précisément si les quantités de graisses formées par lipogénèse deviennent notablement et constamment supérieures à celles qui sont éliminées par lipolyse, il se produit alors dans les adipocytes une accumulation de triglycérides qui, si elle devient excessive, peut se traduire progressivement par une déformation de la peau provoquée par l'épaississement de l'hypoderme dans lequel se trouvent les adipocytes. La surface de la peau devient irrégulière ("peau d'orange") et de consistance plus ou moins flasque ou gélatineuse, donnant finalement à la silhouette un aspect général disgracieux pouvant évoluer entre la simple surcharge locale (lipodismorphie), en passant par l'embonpoint certain, et enfin la réelle obésité.

Or, compte tenu notamment du profond inconfort tant physique qu'esthétique, et parfois psychologique, qu'elle occasionne auprès des individus qui en sont atteints, l'adiposité constitue de nos jours un trouble de moins en moins bien supporté ou accepté.

Des solutions ont été proposées dans l'art antérieur pour intervenir sur le métabolisme des acides gras qui est l'une des cibles privilégiées dans le contrôle de cette surcharge lipidique adipocytaire.

Celui-ci peut être modulé :

- soit par blocage du transport du glucose à l'intérieur de l'adipocyte qui conduit à une diminution des acides gras entrant dans l'adipocyte,
- soit par inhibition de la lipoprotéine lipase,
- soit par activation de la triglycéride lipase (ou lipase hormonosensible), généralement en stimulant l'AMP cyclique, généralement par activation de l'adényl cyclase, ou en provoquant son accumulation par inhibition de la phosphodiestérase.

D'autres voies biologiques ont été explorées pour agir sur le mécanisme de la lipogénèse et/ou de la lipolyse. Il a ainsi été proposé d'utiliser des antagonistes des récepteurs du neuropeptide Y (NPY), qui est un neuromédiateur intervenant dans un certain nombre de processus physiologiques et dont l'implication dans la régularisation de la lipolyse a pu être démontrée (P. Valet, J. Clin. Invest., 1990, 85, 291-295). On peut également utiliser des antagonistes des récepteurs $\alpha_2$ ou encore des agonistes des récepteurs $\beta$3-adrénergiques.

Les compositions cosmétiques proposées jusqu'à présent en vue de traiter l'adiposité contiennent ainsi des composés dits amincissants qui agissent sur un ou plusieurs des mécanismes mentionnés précédemment. Parmi ceux-ci, on peut plus particulièrement citer les bases xanthiques (i.e. des dérivés de la xanthine), telles que la théophylline, la caféine, la théobromine et les 1-hydroxyalkylxanthines et leurs sels compatibles (voir le document FR-A-2 617 401), qui sont des inhibiteurs de phosphodiestérase, les dérivés de l'acide nicotinique tels que plus particulièrement le nicotinate d'alpha-tocophérol et le nicotinate d'hexyle (voir le document EP-A-371 844), les substances dites $\alpha_2$ bloqueurs capables de bloquer les récepteurs $\alpha_2$ à la surface des adipocytes comme par exemple le ginkgo biloba (voir le document FR-A-2 669 537 ou US 5,194,259), et les agonistes des récepteurs $\beta$3-adrénergique tels que l'alvérine et ses sels.

La demande WO 00/30603 décrit des compositions amincissantes contenant un extrait de Dioscorea opposita. Le document FR 2819409 concerne également l'utilisation de diosgénine, pouvant être obtenue par synthèse ou extraite de Disocorea opposita, dans des compositions amincissantes, ce composé diminuant l'accumulation de triglycérides dans l'adipocyte par compétitivité.

US 6,399,089 décrit des compositions pour réguler le poids corporel par inhibition de la recapture de la sérotonine et inhibition de la lipogénèse, ces compositions pouvant contenir de la quercétine, du cacao, du chrome et des extraits d'Hypericum perforatum, de Garcinia cambodgia, de Ginkgo biloba et de Panax ginseng, l'ensemble de ces composés devant être présents.

WO 01/64177 concerne l'utilisation, pour le traitement de la cellulite, de flavones, d'isoflavones ou de leurs glycosides; ces composés sont apportés notamment sous forme d'extraits de soja. FR 2578165 décrit également l'utilisation de bioflavonoïdes, en association avec des teintures d'Hedra helix et de Fucus vesiculorum, dans des pommades anti-cellulitiques.

FR 2774589 propose un mélange de mucopolysaccharidase et d'escine dans des compositions cosmétiques amincissantes; l'escine a pour rôle de stimuler la lipolyse en masquant les récepteurs β-adrénergiques de l'adipocyte.

WO 02/089758 décrit l'utilisation d'oligomères procyanidoliques (ou OPC), notamment extraits de Vitis vinifera, pour des compositions de soin de la peau à visée anti-âge.

FR 2790645 concerne des compléments alimentaires comprenant un extrait de raisin riche en polyphénols, visant à inhiber l'émulsification des lipides en milieu gastrique.

WO 92/15293 se rapporte au traitement ou à la prévention de la cellulite par des rétinoïdes.

US 2002/0106388 concerne des méthodes de traitement de la cellulite par des formulations permettant de bloquer les récepteurs aux oestrogènes, d'inhiber la destruction du collagène et de stimuler la lipolyse; les compositions contiennent un mélange d'isoflavones telles que génistéine ou quercétine, de dérivés de la carnitine, de bases xanthiques telles que la théophylline et d'extrait de Coleus forskolii. Ces différentes solutions peuvent donner de bons résultats, mais il existe toujours un besoin de disposer de nouveaux moyens de lutter contre les surcharges pondérales inesthétiques, en particulier les lipodystrophies localisées, en vue d'obtenir un effet général, ou au contraire localisé, d'amincissement et/ou d'affinement du corps ou du visage.

A cet égard, la disponibilité des précurseurs de la lipogénèse dans l'adipocyte constitue un facteur de régulation important. En effet, il faut éviter que les métabolites générés par la lipolyse ne soient immédiatement ré-utilisés par l'adipocyte pour stocker des triglycérides.

Dans le cadre de la présente invention, il a maintenant été trouvé qu'il est possible d'agir en favorisant l'élimination de l'un de ces précurseurs par l'adipocyte.

C'est pourquoi la présente invention a pour objet l'utilisation d'au moins un modulateur de l'aquaglycéroporine adipeuse (AQPap) dans une composition cosmétique en tant qu'agent amincissant pour diminuer le volume des adipocytes: selon l'invention, ledit modulateur est un agent stimulant de l'activité AQPap des adipocytes et on utilise une association d'au moins (i) un modulateur augmentant l'activité intrinsèque de l'AQPap choisi parmi les extraits de poudre de cacao, les sapogénines de Dioscorea opposita et le lycopène encapsulé sous forme de nanocapsules et (ii) un modulateur augmentant la synthèse d'AQPap choisi parmi l'escine, les complexes de phospholipides et de proanthocyanidines d'écorce de marron d'Inde, et les rétinoïdes.

Les aquaporines sont des protéines membranaires (canaux) assurant le passage de l'eau et de petites molécules comme l'urée et le glycérol, appartenant à la famille des MIP (major intrinsic protein). Elles ont été identifiées sur les cellules, aussi bien dans différents organes chez les mammifères que dans des plantes ou des organismes procaryotes. On distingue fonctionnellement 2 sous-groupes: les aquaporines qui sont sélectives pour l'eau ou l'urée, et les aquaglycéroporines, qui transportent également le glycérol.

Environ 10 types d'aquaporines (aussi notées AQP dans ce qui suit) ont été identifiées dans les tissus de mammifères, telle que AQP1 dans les érythrocytes, AQP2, AQP3 et AQP6 dans le rein, AQP4 dans le cerveau, AQP5 dans les glandes lacrymales et salivaires, AQP9 dans le foie. Une aquaporine spécifique du tissu adipeux a été caractérisée par Kuriyama et al (Biochem. Biophys. Res. Commun., 1997, 241, 53-58) : il s'agit d'une aquaglycéroporine, appelée AQPap et elle présente des homologies avec l'AQP7 du testicule de rat. L'expression du gène de l'AQPap est réprimée par l'insuline (Kishida et al, 2001, J.Biol.Chem., 276, 36251-36260).

L'utilisation d'aquaporines ou de modulateurs d'aquaporines a été proposée en cosmétique pour favoriser l'hydratation de la peau par exemple dans FR2831058; la demande WO01/37799 propose des extraits d'Ajuga Turkenistanica pour réguler les aquaporines 3 et assurer une meilleure hydratation de l'épiderme. Par ailleurs, la demande EP1084700 décrit des compositions anti-transpirantes contenant une quantité efficace d'un modulateur d'aquaporine.

Cependant, il n'a jamais été proposé à ce jour d'utiliser des modulateurs d'aquaporines en tant qu'agent amincissant.

En accord avec l'invention, les modulateurs d'AQPap provoquent la diminution du volume des adipocytes, d'une part en limitant la re-synthèse immédiate de triglycérides à partir du glycérol libéré, d'autre part en réduisant le contenu en glycérol et en eau de ces cellules. Par modulateurs d'AQPap selon l'invention on entend de préférence un

agent capable de stimuler l'activité AQPap des adipocytes, plus spécifiquement des adipocytes matures. Avantageusement, le modulateur d'AQPap augmente la sortie de glycérol des adipocytes.

Selon l'un des aspects de l'invention, les modulateurs d'AQPap augmentent la synthèse d'aquaglycéroporine par les adipocytes. Selon un autre de ses aspects, le modulateur augmente la sortie de glycérol des adipocytes, en favorisant l'ouverture des canaux AQPap, en augmentant leur nombre et/ou en favorisant leur translocation vers la membrane, ou via l'ensemble de ces mécanismes.

Le modulateur d'AQPap pourra être introduit dans la composition sous forme purifiée, notamment par une molécule de synthèse, ou sous forme d'un extrait, par exemple végétal ou bactérien, contenant un ou plusieurs agents modulateur d'AQPap, éventuellement associé à d'autres composants.

En particulier, le modulateur peut être choisi parmi les extraits de poudre de cacao titrés en polyphénols et en oligomères proanthocyanidiques, tel que le produit Caophénol® commercialisé par la société Solabia, l'escine, les proantocyanidines de marron d'Inde, les complexes de phospholipides et de proanthocyanidines d'écorce de marron d'Inde notamment le produit commercialisé comme anti-âge sous la dénomination PA2 Affilène® par la société Indena, les sapogénines telles que la diosgénine, et en particulier des extraits de tubercule de Dioscorea opposita (ou yam) titrés en diosgénine commercialisés sous la dénomination Dioschol® par la société Sederma, les extraits de sauge et notamment les extraits de sauge fermenté, l'acide (tridec-2-ynylthio)acétique, l'acide {[4-(octylthio)but-2-ynyl]thio}acétique

De préférence, les modulateurs utiles selon l'invention induisent, quand ils sont introduits dans une culture d'adipocytes matures, une augmentation de la synthèse d'AQPap, en particulier du gène d'AQPap, par ces adipocytes supérieure ou égale à 1,5 fois, par rapport à une culture d'adipocytes matures en absence de modulateur d'AQPap.

Des modulateurs d'AQPap adaptés à la mise en oeuvre de l'invention pourront ainsi être sélectionnés par un procédé comportant les étapes suivantes:

a) on réalise des cultures d'adipocytes matures différenciés (i) en présence du produit à tester et (ii) en absence du produit à tester,

b) après incubation, on récolte les cellules cultivées selon (i) ou (ii)

c) on extrait l'ARN total et on compare l'expression du gène correspondant à l'AQPap dans les cellules cultivées en présence (i) ou en absence (ii) du produit à tester

d) on sélectionne le produit pour lequel l'expression de l'AQPap dans les adipocytes cultivés en sa présence est augmentée d'un facteur d'au moins 1,5 par rapport à la culture effectuée sans le produit à tester.

Avantageusement, la comparaison de l'expression du gène dans les deux types de culture, à l'étape c) est effectuée par des techniques mettant en oeuvre la RT-PCR quantitative.

Alternativement, on sélectionne un modulateur d'AQPap selon l'invention par un procédé dans lequel les étapes a) et b) sont similaires à ce qui précède, et on évalue ensuite la quantité de la protéine AQPap présente dans les cellules soit après extraction, par marquage par des techniques connues de l'homme du métier comme le "western blot", soit par visualisation sur la culture fixée, par exemple par immunofluorescence. Comme précédemment, on sélectionnera les produits pour lesquels la quantité d'AQPap dans les adipocytes provenant des cultures (i) en présence du produit est supérieure ou égale à 1,5 fois la quantité d'AQPap dans les cultures (ii).

Les modulateurs sélectionnés par le procédé selon l'invention correspondent à des agents augmentant le nombre de canaux AQPap.

D'autres actifs modulateurs d'AQPap utiles selon l'invention pourront être mis en évidence par un procédé comportant les étapes consistant à

- mettre en culture des adipocytes différenciés, matures, en présence ou en absence du produit à tester,
- comparer la quantité de glycérol présente dans le surnageant de culture après incubation dans les cultures effectuées en présence du produit à tester à la quantité de glycérol présente dans le surnageant de culture après incubation sans ajout du produit à tester,
- sélectionner le produit pour lequel la quantité de glycérol libéré dans le surnageant de culture des adipocytes est augmentée d'au moins 1.2 fois, et notamment d'au moins 1,5 fois, par rapport à la quantité de glycérol dans le surnageant de culture d'adipocytes matures n'ayant pas reçu de modulateur d'AQPap.

Les produits ainsi sélectionnés sont des activateurs d'AQPap utilisables selon l'invention.

Ces modulateurs d'AQPap induisent la diminution du volume des adipocytes, et donc du tissu graisseux, en favorisant l'élimination du glycérol provenant notamment de la lipolyse physiologique. Ils peuvent par ailleurs avoir en outre une activité lipolytique intrinsèque, et donc une activité amincissante combinant différents mécanismes.

Selon l'un des aspects de l'invention, le modulateur d'AQPap favorise également la sortie des acides gras libres non estérifiés (ou AGNE) de l'adipocyte. A partir de la lipolyse, et donc clivage des triglycérides en AGNE et glycérol, on observe pour certains modulateurs, lorsque le fonctionnement du canal AQPap est inhibé, par exemple par la présence

d'un sel de mercure, que le glycérol ne sort plus de la cellule adipocytaire, démontrant que la libération de glycérol passe par les canaux AQap; en revanche, les AGNE sont toujours excrétés par la cellule, par une autre voie que les AQPap.

Selon l'invention, on utilise une association d'au moins un modulateur susceptible d'augmenter le nombre des canaux AQPap et d'au moins un modulateur d'AQPap susceptible de stimuler l'activité intrinsèque des canaux AQPap. Par agent stimulant de l'activité intrinsèque des canaux AQPap on entend un agent qui favorise la translocation des canaux AQPap vers la membrane de l'adipocyte et/ou qui favorise l'ouverture desdits canaux AQPap (et donc le passage du glycérol par les canaux AQPap.

L'agent susceptible d'augmenter le nombre des canaux AQPap est en particulier un agent capable d'augmenter l'expression et/ou la synthèse de l'AQPap.

Selon l'un des aspects de l'invention, un même agent remplit les deux fonctions en augmentant le nombre des canaux AQPap et en stimulant leur activité intrinsèque.

Selon un autre de ses aspects, on utilise une association d'au moins deux modulateurs différents, dont les fonctions au niveau des activités sur les canaux aquaporines seront au moins partiellement, complémentaires.

En particulier, dans de telles associations, l'agent modulateur capable d'augmenter l'activité de canaux aquaporine adipose (AQPap) pourra être choisi dans le groupe comprenant les sapogénines de Dioscorea opposita telles que la diosgénine, et en particulier le Dioschol®, le lycopène encapsulé sous forme de nanocapsules, notamment de polycaprolactone contenant un extrait de tomate à 0,5% enrichi en lycopène, et les extraits de poudre de cacao titrés en polyphénols et en oligomères proanthocyanidiques, tel que le produit Caophénol® ; l'agent modulateur capable d'augmenter l'expression de l'aquaporine et/ou le nombre de canaux AQPap pourra être choisi parmi le lycopène encapsulé sous forme de nanocapsules, notamment de polycaprolactone contenant un extrait de tomate à 0,5% enrichi en lycopène, l'escine, les complexes de phospholipides et de proanthocyanidines d'écorce de marron d'Inde tels que le produit commercialisé sous la dénomination PA2 Affilène®, les extraits de sauge, l'acide (tridec-2-ynylthio)acétique et l'acide{[4-(octylthio)but-2-ynyl]thio)acétique et les rétinoïdes.

Le lycopène encapsulé tel que défini ci-dessus est par exemple un agent modulateur susceptible de remplir les deux fonctions, c'est-à-dire d'augmenter le nombre de canaux AQPap et de stimuler l'activité intrinsèque des canaux AQPap.

Avantageusement, de tels modulateurs seront utilisés dans les compositions selon l'invention, en association avec au moins un autre agent amincissant, en particulier au moins un autre agent modulateur de l'AQPap tel que défini dans ce qui précède.

La quantité de modulateur d'AQPap présent dans la composition sera adaptée par l'homme du métier selon le type de produit pour obtenir une quantité efficace cosmétologiquement, notamment s'il s'agit d'une molécule purifiée ou d'un extrait végétal ou cellulaire le contenant. A titre indicatif, la concentration de modulateur présent dans la composition pourra varier de 0,001 à 20% en poids par rapport au poids total de la composition, notamment de 0,01 à 10%. Ainsi, des compositions adaptées à la mise en oeuvre de l'invention contiendront par exemple une association de 0,05 à 0,5% d'escine, et de 0,5 à 5% de Dioschol®, sans que ces concentrations puissent s'entendre comme limitant l'invention.

Selon l'un des modes de réalisation de l'invention, le modulateur d'AQPap est présent dans une composition qui peut contenir contient en outre au moins un agent amincissant lipolytique et/ou inhibiteur de la lipogenèse et/ou inhibiteur de la différenciation adipocytaire. L'action lipolytique et/ou amincissante de ces agents sera renforcée par l'activité du modulateur d'AQPap, qui facilitera l'élimination du glycérol produit par la lipolyse. L'agent lipolytique peut notamment être choisi parmi :

- les inhibiteurs de phosphodiestérase
  on peut citer en particulier les bases xanthiques et les extraits naturels en contenant. Des exemples sont constitués par la caféine et ses dérivés, notamment les 1-hydroxyalkylxanthines décrites dans le document FR-A-2 617 401, le citrate de caféine, la théophylline et ses dérivés, la théobromine, l'acéfylline, l'aminophylline, le chloroéthylthéophylline, le diprophylline, le diniprophylline, l'étamiphylline et ses dérivés, l'étofylline et la proxyphylline. Ces bases xanthiques peuvent être utilisées seules ou dans des mélanges commerciaux tels que l'association de caféine et d'alginate et caféinate de méthylsilanetriol commercialisé par la société EXSYMOL sous la dénomination commerciale Caféisilane C. Comme extraits naturels contenant des bases xanthiques, on peut citer les extraits de thé, de café, de guarana, de maté, de cola (*Cola Nitida*) et notamment l'extrait sec de fruit de guarana (*Paulina sorbilis*) contenant de 8 à 10% de caféine. On peut aussi citer le Ma Huang (Ephedra plant) qui contient de l'éphédrine.
- les composés α-2 bloqueurs capables de bloquer les récepteurs α-2 à la surface des adipocytes,
- les agonistes β-adrénergiques,
  comme agoniste β-adrénergique, on peut citer en particulier l'alvérine ou un sel organique ou inorganique d'alvérine tel que le citrate d'alvérine.
- les composés inhibant la synthèse des récepteurs aux LDL ou VLDL,
- les inhibiteurs des enzymes de la synthèse des acides gras,
  tels que l'acétyl CoA carboxylase ou la fatty acid synthetase et en particulier la cérulénine.
- les composés stimulant les récepteurs β et/ou les protéines G,

- les bloqueurs du transport du glucose,
  tels que la sérine ou la rutine.
- les antagonistes du neuropeptide Y (NPY) capable de bloquer les récepteurs NPY à la surface des adipocytes,
- les agents modifiant le transport des acides gras,
- les peptides lipolytiques et les protéines lipolytiques,

comme des peptides ou protéines tels que les peptides dérivés de l'hormone parathyroïdienne, décrits notamment dans les brevets FR-2 788 058 et FR-2 781 231 ou les peptides décrits dans le document FR-2 786 693, sans que cette liste soit limitative. On peut citer également les protamines et leurs dérivés tels que ceux décrits dans le document FR-2 758 724 et les peptides natriurétiques.
D'autres exemples d'agents lipolytiques utilisables sont certains extraits végétaux ne contenant pas de caféine et certains extraits d'origine marine, on peut citer en particulier :

- comme extraits végétaux : les extraits de *Garcinia cambogia,* de *Bupleurum chinensis,* de lierre grimpant *(Hedera helix),* d'arnica (*Arnica montana L.*), de romarin (*Rosmarinus officinalis N.*), de souci (*Calendula officinalis*), de sauge (*Salvia officinalis L.*), de ginseng (*Panax ginseng*), de millepertuis (*Byperycum perforatum*), de fragon *(Ruscus aculeatus L.),* d'ulmaire (*Filipendula ulmaria L.*), d'orthisiphon (*Orthosiphon stamincus benth),* de bouleau (*Betula alba*), de cécropia, d'arganier, de *Ginkgo biloba,* de prêle, d'escine, de cangzhu, de *Chrysanthellum indicum,* de dioscorées riche en diosgénine ou hécogénine dont *Dioscorea opposita* ou *mexicana* ou *villosa,* de plantes du genre *Armeniacea, Atractylodis platicodon, sinom-menum, Pharbitidis* ou *Flemingia,* de Coleus tels que *Coleus forskohlii* ou *blumei* ou *esquirolii* ou C. *scutellaroides* ou *xanthantus* ou *barbatus,* tel que l'extrait de racine de *Coleus barbatus* contenant 60% de forskoline, les extraits de ballote, de *Guioa,* de *Davallia,* de *Terminalia,* de *Barringtonia,* de *Trema* et *d'Antirobia ;*
- comme extraits d'origine marine : les extraits d'algue ou de phytoplancton, tels qu'un extrait de Laminaria digitata commercialisé sous la dénomination PHYCOX75 par la société SECMA, l'algue skeletonema telle que décrite notamment dans le brevet FR-2 782 921 ou les diatomées telles que celles décrites dans le brevet FR-2 774 292.

La quantité d'actif lipolytique ou inhibant la lipogénèse, présente dans la composition selon l'invention peut varier dans une large mesure et sera de préférence comprise entre 0,001 et 20 % en poids, mieux, entre 0,1 et 10 % en poids, par rapport au poids total de la composition.
Avantageusement, la composition selon l'invention contient au moins un extrait de Dioscorée riche en diosgénine, par exemple provenant de racines d'igname sauvage. On pourra par exemple choisir un extrait de racines de *Dioscorea opposita* commercialisé en solution dans un dérivé de polyéthylèneglycol (6OE) et de mélange de mono-, di-, et triglycéride d'acides caprylique et caprique / conservateurs / glycérine (rapport pondéral 1/93,8/0,2/5), commercialisé sous la dénomination Dioschol par la société Sederma.
Selon un mode de réalisation avantageux la composition contient en outre au moins un agent choisi parmi les agents desquamants, les agents raffermissants, les agents promoteurs de synthèse des constituants de la matrice extracellulaire ou inhibiteurs de leur dégradation, les agents hydratants, les modulateurs d'aquaporines, les agents actifs sur la microcirculation, les agents actifs sur le métabolisme énergétique des cellules.
On peut citer, de manière non limitative

- les actifs agissant sur la microcirculation (vasculoprotecteurs ou vasodilatateurs) tels que les flavonoïdes, les ruscogénines, les esculosides naturels ou de synthèse (dont le Perméthol commercialisé par la société SOCHIBO), l'escine extrait du marron d'Inde, les nicotinates, l'hépéridine méthyl chalcone, le petit houx, les huiles essentielles de lavande ou de romarin, les extraits de *Ammi visnaga* ;les donneurs de NO ou les modulateurs directs ou indirects de nitric oxyde synthase.
- les actifs raffermissants et/ou anti-glycants (empêchant la fixation du sucre sur les fibres de collagène) tels que les extraits de *Centella asiatica* et de *Siegesbeckia,* le silicium, l'amadorine, l'ergothionéine et ses dérivés, les hydroxystilbènes et leurs dérivés, notamment le resvératrol, les extraits végétaux de la famille des Ericaceae, notamment les extraits de myrtille (*Vaccinium angustifollium*), la vitamine C et ses dérivés et le rétinol et ses dérivés.

Par "agent desquamant", on entend tout composé capable d'agir :

- soit directement sur la desquamation en favorisant l'exfoliation, tel que les $\beta$-hydroxyacides, en particulier l'acide salicylique et ses dérivés (dont l'acide n-octanoyl 5-salicylique) ; les $\alpha$-hydroxyacides, tels que les acides glycolique, citrique, lactique, tartrique, malique ou mandélique ; l'urée ; l'acide gentisique ; les oligofucoses ; l'acide cinnamique ; l'extrait de Saphora japonica ; le resvératrol ;
- soit sur les enzymes impliquées dans la desquamation ou la dégradation des cornéodesmosomes, les glycosidases,

la stratum corneum chymotryptic enzym (SCCE) voire d'autres protéases (trypsine, chymotrypsine-like). On peut citer les agents chélatant des sels minéraux : l'EDTA ; l'acide N-acyl-N,N',N' éthylène diaminetriacétique ; les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine (tels que décrits dans EP-0 852 949, ainsi que le méthyl glycine diacétate de sodium commercialisé par BASF sous la dénomination commerciale TRILON M) ; le miel ; les dérivés de sucre tels que l'O-octanoyl-6-D-maltose et la N-acétyl glucosamine.

Les agents agissant sur le métabolisme énergétique des cellules cutanées tel que, par exemple, et de façon non limitative, la synthèse d'ATP, ceux qui interviennent sur la chaine respiratoire de la cellule ou sur les réserves énergétiques. On peut citer le Coenzyme Q10 (ubiquinone), le cytochrome C, la créatine ou encore la phosphocréatine.

Des modulateurs d'aquaporines différentes de l'AQPap pourront avantageusement être présents dans les compositions selon l'invention, en particulier des aquaporines impliquées dans le transport d'eau, comme AQP 3 ou 4, de façon à éviter la formation d'oedème et favoriser le drainage du tissu adipeux. On utilise par exemple des extraits végétaux contenant de tels modulateurs, comme par exemple les extraits cités dans WO 0137799, ou les composés modifiant le cytosquelette cité dans WO0164219 de Janssen Pharm, ou des composés pouvant se lier à la lipocalin (WO0112225, SANTEN) ou l'interferon alpha.

Les agents promoteurs des synthèse des constituants de la matrice extracellulaire sont notamment des agents stimulant la synthèse des macromolecules du derme ou de l'épiderme ou empêchant leur dégradation. Parmi les actifs stimulant les macromolécules du derme ou empêchant leur dégradation, on peut citer ceux qui agissent :

- soit sur la synthèse du collagène, tels que les extraits de Centella asiatica ; les asiaticosides et dérivés ; l'acide ascorbique ou vitamine C et ses dérivés ; les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine® ; et les hormones végétales telles que les auxines et les lignanes.
- soit sur la synthèse d'élastine, tels que l'extrait de Saccharomyces Cerivisiae commercialisé par la société LSN sous la dénomination commerciale Cytovitin® ; l'extrait d'algue Macrocystis pyrifera commercialisé par la société SECMA sous la dénomination commerciale Kelpadelie®
- soit sur la synthèse des glycosaminoglycanes, tels que le produit de fermentation du lait par lactobacillus vulgaris, commercialisé par la société BROOKS sous la dénomination commerciale Biomin yogourth® ; l'extrait d'algue brune Padina pavonica commercialisé par la société ALBAN MÜLLER sous la dénomination commerciale HSP3® ; l'extrait de Saccharomyces cerevisiae disponible notamment auprès de la société SILAB sous la dénomination commerciale Firmalift® ou auprès de la société LSN sous la dénomination commerciale Cytovitin® ; des dérivés de xylose comme l'Aquaxyl®; et des dérivés C-glycosides tels que ceux décrits dans la demande EP1345919, en particulier le C-β-D-xylopyranoside-2-hydroxy-propane et ses sels physiologiquement acceptables;
- soit sur la synthèse de la fibronectine, tels que l'extrait de zooplancton Salina commercialisé par la société SEPORGA sous la dénomination commerciale GP4G® ;
  l'extrait de levure disponible notamment auprès de la société ALBAN MÜLLER sous la dénomination commerciale Drieline® ; et le palmitoyl pentapeptide commercialisé par la société SEDERMA sous la dénomination commerciale Matrixil® ;
- soit sur l'inhibition des métalloprotéinases (MMP) telles que plus particulièrement les MMP 1, 2, 3, 9 . On peut citer : les rétinoïdes et dérivés, les oligopeptides et les lipopeptides, les lipoaminoacides, l'extrait de malt commercialisé par la société COLETICA sous la dénomination commerciale Collalift® ; les extraits de myrtille ou de romarin ; le lycopène ; les isoflavones, leurs dérivés ou les extraits végétaux en contenant, en particulier les extraits de soja (commercialisé par exemple par la société ICHIMARU PHARCOS sous la dénomination commerciale Flavostérone SB®), de trèfle rouge, de lin, de kakkon ou de sauge ;
- soit sur l'inhibition des sérine protéases telles que l'élastase leucocytaire ou la cathepsine G. On peut citer : l'extrait peptidique de graines de légumineuse (Pisum sativum) commercialisé par la société LSN sous la dénomination commerciale Parelastyl®; les héparinoïdes ; les pseudodipeptides tels que l'acide {2-[acétyl-(3-trifluorométhyl-phényl)-amino]-3-méthyl-butyrylamino} acétique; et les inhibiteurs d'élastase tels que les N-acyl-aminoamides tels que décrits dans la demande EP 1 292 608, en particulier l'acide {2-[acétyl-(3-trifluorométhyl-phényl)-amino]-3-méthyl-butyrylamino} acétique, le {2-[acétyl-(3-trifluorométhyl-phényl)-amino]-3-méthyl-butyrylamino} acétate d'éthyle, l'acide [2-(acétyl-benzyl-amino)-3-méthyl-butyrylamino] acétique, le [2-(acétyl-benzyl-amino)-3-méthyl-butyrylamino] acétate d'éthyle, le (2-{benzyl-[(diethoxy-phosphoryl)-acétyl]-amino}-3-méthyl-butyrylamino) acétate d'éthyle;

Parmi les actifs stimulant les macromolécules épidermiques, telles que la fillagrine et les kératines, on peut citer notamment l'extrait de lupin commercialisé par la société SILAB sous la dénomination commerciale Structurine® ;

l'extrait de bourgeons de hêtre Fagus sylvatica commercialisé par la société GATTEFOSSE sous la dénomination commerciale Gatutine® ; et l'extrait de zooplancton Salina commercialisé par la société SEPORGA sous la dénomination commerciale GP4G®.

Les compositions pourront également contenir des agents stimulateurs de bronzage, régulateurs de la production de sébum ou des agents photoprotecteurs.

Là encore, la quantité de ces actifs complémentaires peut varier dans une large mesure. De préférence, ces actifs seront présents dans la composition selon l'invention en quantité représentant de 0,01 à 15 % et, mieux, de 0,05 à 10 % en poids, par rapport au poids total de la composition.

Selon l'un des modes de réalisation de l'invention, les compositions sont essentiellement exemptes de chrome ou de ses sels tels que le picolinate de chrome, c'est-à-dire que les quantités éventuellement présentes sont inférieures à celles permettant d'obtenir un effet sur l'amincissement.

Selon encore un mode de réalisation, les compositions selon l'invention sont essentiellement dépourvues de carnitine ou de ses dérivés, c'est-à-dire que les quantités éventuellement présentes sont inférieures à celles permettant d'obtenir un effet sur l'amincissement et/ou la lipolyse.

Selon l'un des modes de réalisation de l'invention, le modulateur d'AQPap est utilisé dans des compositions à usage topique externe.

Elle peut notamment être sous forme de solution aqueuse, alcoolique, hydro-alcoolique ou huileuse, de suspension, de dispersion, d'émulsions E/H, H/E ou multiple, de gels aqueux ou anhydres, de dispersion vésiculaires de type ionique ou non ionique. Elle peut avoir une consistance solide, semi-liquide, pâteuse, ou solide.

Pour une application topique, la composition utilisable selon l'invention peut être notamment sous la forme d'une solution aqueuse, hydroalcoolique ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H) ou multiples, d'une poudre libre ou compactée à utiliser telle quelle ou à incorporer dans un milieu physiologiquement acceptable, ou de suspensions ou émulsions de consistance molle du type crème ou gel aqueux ou anhydres, ou encore de microcapsules ou microparticules, ou de dispersions vésiculaires de type ionique et/ou non ionique. Elle peut ainsi se présenter sous forme d'onguent, de teinture, de crème, de pommade, de poudre, de timbre, de tampon imbibé, de solution, d'émulsion ou de dispersion vésiculaire, de lotion, de gel, de spray, de suspension, de shampooing, d'aérosol ou de mousse. Elle peut être anhydre ou aqueuse. Elle peut également consister en des préparations solides constituant des savons ou des pains de nettoyage.

Ces compositions sont préparées selon les méthodes usuelles.

Selon un autre mode de réalisation de l'invention, la composition est adaptée à un usage oral, en particulier en "cosmétique orale".

Pour un usage par voie orale, la composition peut notamment se présenter sous forme de capsules, gélules, dragées, de granulés, de comprimés, de pâte à mâcher, de gels ou de sirops buvables ou de toute autre forme connue de l'homme du métier.

Les quantités des différents constituants des compositions utilisables selon l'invention sont celles classiquement utilisées dans les domaines considérés.

La phase aqueuse contient de l'eau et éventuellement un ingrédient miscible en toute proportion à l'eau comme les alcools inférieurs en $C_1$ à $C_8$ tel que l'éthanol, l'isopropanol, les polyols comme le propylène glycol, le glycérol, le sorbitol ou encore l'acétone ou l'éther.

Lorsque la composition utilisable selon l'invention est une émulsion, la proportion de la phase grasse peut aller de 2 à 80%, notamment de 5% à 80% en poids, et de préférence de 5% à 50% en poids par rapport au poids total de la composition. Les huiles, les cires, les émulsionnants et les co-émulsionnants utilisés dans la composition sous forme d'émulsion sont choisis parmi ceux classiquement utilisés dans le domaine cosmétique. L'émulsionnant et le co-émulsionnant sont présents, dans la composition, en une proportion allant de 0,1, notamment de 0,3% à 30% en poids, de préférence de 0,5 à 20% en poids, et mieux de 1 à 8% par rapport au poids total de la composition. L'émulsion peut, en outre, contenir des vésicules lipidiques et notamment des liposomes.

Lorsque la composition utilisable selon l'invention est une solution ou un gel huileux, la phase grasse peut représenter plus de 90% du poids total de la composition.

De façon connue, la composition selon l'invention peut contenir également des adjuvants habituels dans le domaine cosmétique, tels que les gélifiants ou épaississants hydrophiles ou lipophiles, les additifs hydrophiles ou lipophiles, les conservateurs, les antioxydants, les solvants, les parfums, les charges, les filtres, les absorbeurs d'odeur les électrolytes, les neutralisants, les agents bloqueurs d'U.V. (irradiation de rayonnement ultraviolet) comme les filtres solaires, les polymères filmogènes, les actifs cosmétiques et pharmaceutiques à action bénéfique pour la peau ou les fibres kératiniques et les matières colorantes, solubles ou non dans le milieu. Les quantités de ces différents adjuvants sont celles classiquement utilisées dans le domaine cosmétique, et notamment de 0,01 à 50% du poids total de la composition, par exemple de 0,01% à 20%, notamment inférieure ou égale à 10% du poids total de la composition, et en particulier supérieure ou égale à 0,1 %. Ces adjuvants, selon leur nature, peuvent être introduits dans la phase grasse, dans la

phase aqueuse et/ou dans les sphérules, vésicules ou microsphères lipidiques, telles que les liposomes.
La phase grasse peut contenir des composés gras ou huileux, liquides à température ambiante (25°C) et pression atmosphérique (760 mm de Hg), généralement appelés huiles. Ces huiles peuvent être compatibles ou non entre elles et former une phase grasse liquide macroscopiquement homogène ou un système bi- ou triphasique.
La phase grasse peut, en plus des huiles, contenir des cires, des gommes, des polymères lipophiles, des produits "pâteux" ou visqueux contenant des parties solides et des parties liquides.

Comme huiles ou cires utilisables dans l'invention, on peut citer les huiles minérales (huile de vaseline, isoparaffine hydrogénée), les huiles végétales (fraction liquide du beurre de karité, huile de tournesol, de soja, de blé), les huiles animales (perhydrosqualène), les huiles de synthèse (huile de Purcellin, esters d'acides gras), les huiles ou cires siliconées (polydiméthylsiloxanes linéaires ou cycliques, cyclométhicone, phényltriméthicone) et les huiles fluorées (perfluoropolyéthers), les cires d'abeille, de candelila, carnauba ou paraffine. On peut ajouter à ces huiles et cires des alcools gras et des acides gras libres (acide stéarique, linoléique, linolénique).
Comme émulsionnants utilisables dans l'invention, on peut citer par exemple le stéarate ou laurate de glycérol, les stéarates ou oléates de sorbitol, les alkyl diméthiconecopolyol (avec alkyle ≥ 8) et leurs mélanges; le stéarate ou oléate de sorbitol polyoxyéthyléné, par exemple le polysorbate 60 et le mélange de PEG-6/PEG-32/Glycol Stéarate vendu sous la dénomination de Tefose® 63 par la société Gattefosse, le monostéarate ou monolaurate de polyéthylène glycol, les diméthiconecopolyols et leurs mélanges.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les Bentones®, les sels métalliques d'acides gras comme les stéarates d'aluminium et la silice hydrophobe, éthylcellulose, polyéthylène.

Avantageusement, la composition se présente sous forme d'une émulsion eau-dans-huile (E/H), contenant un glycéride d'acide gras, un alcool et un tensioactif siliconé particulier, notamment les diméthicones copolyols vendus sous les dénominations DC 5329, DC 7439-146, DC 2-5695 par la société Dow Corning et en particulier le mélange de polydiméthylsiloxane oxyéthyléné oxypropylèné (18OE/18OP), de cyclopentasiloxane et d'eau (rapport pondéral 10/88/2) commercialisé sous la dénomination DC2 5225 C par la société Dow Corning; KF-6013, KF-6015, KF-6016, KF-6017 par la société Shin-Etsu. L'émulsionnant siliconé peut être présent dans la composition selon l'invention en une quantité comprise entre 0,1 % et 5% en poids par rapport au poids total de la composition, préférentiellement en une quantité comprise entre 0,5 et 3 % en poids. L'émulsionnant siliconé pourra être associé à une cyclométhicone.
En particulier, les compositions contenant du Dioschol contiendront un alcool.

Lorsque le modulateur est utilisé dans une composition à usage oral, celle-ci contient avantageusement en outre des sels de calcium (par exemple le carbonate de calcium) du calcium provenant de produits laitiers de l'alimentation (yaourts, lait , lait en poudre...) par exemple à une dose voisine de 1g/jour (plus avantageux), des modulateurs de canaux calciques , l'acide conjugué linoléique, les modulateurs directs ou indirects de la production de NO, les modulateurs directs ou indirects de nitric oxyde synthase. Tous les dérivés peuvent être associés à des facteurs additionnels comme la leucine ou les acides aminés branchés.
Un autre objet de l'invention est l'utilisation d'au moins un agent choisi parmi l'escine, les proanthocyanidines, le lycopène encapsulé, le Caophénol, la disogénine, le Dioschol®, les extraits de Ginkgo, les extraits de sauge, l'acide (tridec-2-ynylthio)acétique, l'acide {[4-(octylthio)but-2-ynyl]thio}acétique et le PA2 Affilène® (ou complexe de phospholipides et de proanthocyanidines d'écorce de marron d'inde 36%) pour moduler les aquaglycéroporines du tissu adipeux, et donc pour diminuer le volume des adipocytes et du tissu adipeux.

Selon encore un autre de ses aspects, l'invention se rapporte à un procédé de traitement cosmétique destiné à prévenir ou diminuer l'augmentation du volume du tissu adipeux et/ou la formation d'amas graisseux, et/ou un procédé d'amincissement comprenant l'application sur tout ou partie du corps d'une composition contenant au moins un modulateur d'AQPap telle que définie précédemment. L'application pourra s'effectuer notamment sur des zones sujettes à des lipodystrophies, telles que l'abdomen, le haut des cuisses ou des bras, ou sur certaines zones du visage telles que le bas du visage. Cette application pourra avantageusement s'effectuer après un effort physique, ou en complément d'une cure d'amincissement. Elle pourra être répétée dans le temps, plusieurs fois par jour et pendant plusieurs semaines ou plusieurs mois.
Le traitement pourra aussi être effectué en complément d'un traitement de l'obésité.

Les exemples qui suivent sont destinés à illustrer l'invention.

Exemple 1 : Dosage du glycérol libéré

Les effets sur la libération de glycérol ont été testés en présence de tous les produits à l'essai et à l'aide d'une

méthode de dosage par fluorescence. Puis, le dosage de glycérol a été a réalisé sur certains produits sélectionnés à l'aide d'une méthode colorimétrique et en présence ou en l'absence de HgCl2.
La plupart des aquaporines des mammifères sont inhibées par le mercure et ses sels, alors que des inhibiteurs chimiques de diffusion ne sont pas connus (P. Agre et al., J. of Physiology, 2002, 542.1).
Les cellules utilisées étaient des pré-adipocytes de souris de lignée 3T3-L1 (40 000 cellules/puits).
Les cellules ont été cultivées à confluence en milieu de croissance, avec un changement de milieu tous les 2-3 jours (plaques 24 puits).

- Milieu de croissance :

  DMEM (Life technologies 21969035)
  L-glutamine 2 mM (Life technologies 25030024)
  pénicilline 50 UI/ml streptomycine 50 μg/ml (life technologies 15070063)
  amphotéricine B 0.25 μg/ml (Sigma A2942)
  sérum de veau foetal 10 % (v/v Life technologies 10106151)

A forte confluence , les cellules ont été transférées en milieu de différenciation pendant 2 fois 48 heures.

- Milieu de différenciation :

  DMEM (Life technologies 21969035)
  L-glutamine 2mM (Life technologies 25030024)
  pénicilline 50 UI/ml streptomycine 50 μg/ml (life technologies 15070063)
  sérum de veau foetal 10 % (v/v Life technologies 10106151)
  0.5 mM methyl-isobutyl xanthine (IBMX, Sigma 17018)
  5 μg/ml insuline (Sigma I1882)
  1 μM dexaméthazone (Sigma D1756)

Après différenciation, les cellules ont été transférées en milieu DMEM +insuline (5 μg/ml final) pendant 72 heures puis en milieu de croissance (DMEM/10 % de sérum).
Des cultures contrôles ont été réalisées en milieu de croissance 3T3-L1 pendant toute la durée de l'expérience (3T3-L1 non différenciés).

DOSAGE DU GLYCEROL LIBERE

***1. Dosage par fluorescence***

Protocole 1 (sans épinéphrine))

Les cellules différenciées ont été placées en milieu d'essai *(MEM 100 % (v/v), bicarbonate 1,87 mg/ml, glutamine 2 mM, albumine bovine sérique délipidée 0,5% (p/v), pénicilline/streptomycine 25 UI/ml/25μg/ml)* puis traitées par les produits à l'essai ou les références. Après incubation 1 h à 37°C et 5% de $CO_2$, les surnageants de culture ont été prélevés afin de réaliser le dosage du glycérol libéré.
Le glycérol libéré dans le milieu de culture a été dosé de manière indirecte par mesure de la fluorescence du NADH produit dans la séquence réactionnelle suivante (d'après Wieland).

$$\text{Glycérol + ATP} \xrightarrow{\text{glycérokinase}} \text{L- }\alpha\text{-glycérophosphate +ADP}$$

$$\text{L-}\alpha\text{-glycérophosphate + NAD} \xrightarrow{\text{Glycérophosphate déshydrogénase}} \text{diaxyacétone phosphate + NADH + }H^+$$

Des contrôles d'auto-fluorescence et de quenching (absorption de la fluorescence) ont été réalisés afin de déterminer une éventuelle interférence des produits dans ce dosage.

Inhibition du transport de glycérol induite par $HgCl_2$ - Protocole 3

Les cellules différenciées ont été placées en milieu d'essai pendant 24 h. Les cellules ont ensuite été placées en tampon PBS contenant ou non du $HgCl_2$ (30μM final) pendant 5 minutes à 37°C. Après traitement par le $HgCl_2$, les cellules ont été lavées deux fois en PBS puis placées en milieu d'essai contenant ou non les produits à l'essai Les surnageants de culture ont ensuite été prélevés afin de réaliser le dosage du glycérol libéré.

2. Dosage colorimétrique du glycérol libéré

La libération de glycérol par les cellules stimulées a été, dans certains cas, confirmée en utilisant un test colorimétrique (Roche), selon les indications du fournisseur. Cette approche nous a permis d'éviter le problème d'auto-fluorescence de certains produits testés, interférant pour l'analyse.

Témoin:

**Libération du glycérol par 3T3-L1 non différenciés et 3T3-L1 différenciés**

| *Cellules* | *Traitement* | *Glycérol (μM)* | *Δ%* |
|---|---|---|---|
| Pre-adipocytes non-differenciés | Non-stimulé | 13.5 | 35 |
| Pre-adipocytes differenciés | Non-stimulé | 39.0 | 100 |
| Pre-adipocytes differenciés | Epinephrine 40 μM | 149.5 | 383 |

L'épinéphrine est un contrôle positif de la lipolyse et stimule l'ouverture des canaux AQPap ainsi que la translocation des AQPap du cytosol vers la membrane cellulaire.

Produits testés:

**Dioschol®**

| **Dosage du glycérol libéré par les cellules 3T3-L1 différenciées**<br>Protocole 1 | | | | |
|---|---|---|---|---|
| **Traitement** | **Glycérol (μM)** | sd | **% témoin** | *ctrl quenching* (%) |
| **Témoin** | **33.41** | 5.67 | **100** | *0* |
| **Epinéphrine 40μM** | **117.57** | 10.90 | **352** | *0* |
| **Dioschol**<br>**0.016 %**<br>**0.0032 %**<br>**0.0006 %** | <br>**126.50**<br>**55.04**<br>**40.77** | <br>7.22<br>1.20<br>1.44 | <br>**346**<br>**162**<br>**122** | <br>*0*<br>*0*<br>*0* |

| **Dosage du glycérol libéré par les cellules 3T3-L1 différenciées**<br>Protocole $HgCl_2$ | | |
|---|---|---|
| **Traitement** | **Glycérol (μM)** | **% témoin** |
| *Contrôle sans Epi* | *34.31* | *40* |
| *Contrôle sans $HgCl_2$* | *160.26* | *185* |
| **Témoin $HgCl_2$** | **86.61** | **100** |
| **Dioschol 0.016 %** | **127.03** | **106** |

(suite)

| **Dosage du glycérol libéré par les cellules 3T3-L1 différenciées** Protocole $HgCl_2$ | | |
|---|---|---|
| **Traitement** | **Glycérol (μM)** | **% témoin** |
| **0.0032 %** | **92.55** | **92** |
| **0.0006 %** | **76.65** | **89** |

**Lycopène encapsulé**

| **Dosage colorimétrique du glycérol libéré par les cellules 3T3-L1 différenciées** Protocole 1 | | |
|---|---|---|
| **Traitement** | **Glycérol (μM)** | **% témoin** |
| **Témoin** | **56.57** | **100** |
| **Epinéphrine 40μM** | **449.74** | **795** |
| **Lycopène encapsulé** | | |
| **0.2 %** | **51.28** | **91** |
| **0.04%** | **59.22** | **105** |
| **0.008%** | **67.97** | **120** |

| **Dosage colorimétrique du glycérol libéré par les cellules 3T3-L1 différenciées** Protocole $HgCl_2$ | | |
|---|---|---|
| **Traitement** | **Glycérol (μM)** | **% témoin** |
| *Contrôle sans Epi* | *38.46* | *40* |
| *Contrôle sans $HgCl_2$* | *413.72* | *432* |
| **Témoin $HgCl_2$** | **95.85** | **100** |
| **Lycopène encapsulé** | | |
| **0.2 %** | **94.42** | **99** |
| **0.04%** | **91.58** | **96** |
| **0.008%** | **85.67** | **89** |

**Caophénol®**

| **Dosage du glycérol libéré par les cellules 3T3-L1 différenciées** Protocole 1 | | | | |
|---|---|---|---|---|
| **Traitement** | **Glycérol (μM)** | **% témoin** | *ctrl quenching* (%) | *% théorique tenant compte du contrôle quenching* |
| **Témoin** | **31.15** | **100** | *0* | *100* |
| **Epinéphrine 40μM** | **101.85** | **327** | *0* | *327* |
| **Caophénol** | | | | |
| **2 %** | **33.31** | **107** | *79* | *510* |
| **0.4 %** | **39.63** | **127** | *53* | *270* |
| **0.08%** | **28.28** | **91** | *22* | *115* |

| **Dosage du glycérol libéré par les cellules 3T3-L1 différenciées** Protocole $HgCl_2$ | | | | |
|---|---|---|---|---|
| **Traitement** | **Glycérol (μM)** | **% témoin** | *ctrl quenching* (%) | *% théorique tenant compte du contrôle quenching* |
| *Contrôle sans Epi* | *29.71* | *34* | *0* | *34* |
| *Contrôle sans $HgCl_2$* | *139.17* | *159* | *0* | *159* |
| **Témoin $HgCl_2$** | **87.71** | **100** | *0* | *100* |
| **βmercaptol 10mM** | **135.74** | **155** | *0* | *155* |
| **Caophénol 2 %** | **30.78** | **35** | *78* | *159* |
| **0.4%** | **61.43** | **70** | *63* | *189* |
| **0.08%** | **57.62** | **66** | *20* | *100* |

Les résultats sont résumés dans le tableau suivant:

| **Nom** | **Relargage de glycerol** |
|---|---|
| Dioschol 0.0006-0.016% | OUI |
| Mexoryl SAQ®: Lycopène encapsulé 0.008-0.2% | OUI |
| Caophénol 0.08-2% | OUI |
| (tridec-2-ynylthio)acetic acid 0.16-4.0μg/ml | Non |
| {(4-(octylthio)but-2-ynyl]thio}acetic acid 0.32-8.0μg/ml | Non |
| Lierre 0.016-0.4% | Non |

Le lierre, connu comme amincissant, n'est pas actif sur les canaux AQPap

Nous observons une libération de glycérol stimulée par le Dioschol, le lycopène encapsulé et le caophénol. En présence de chlorure mercurique, bloquant les canaux AQPap, aucune sortie de glycérol n'est observée, même en présence des actifs. Cela démontre que la libération de glycérol observée passe par les canaux aquaglycéroporines adipocytaires.

Exemple 2: Mesure de l'augmentation de l'expression relative de l'AQPap

L'essai a été réalisé sur des cellules 3T3-L1 différenciées obtenues comme décrit à l'exemple 1. Les cellules différenciées ont été préalablement placées en milieu d'essai* pendant 24 heures puis traitées par les produits à l'essai ou les références. Après incubation 6 h à 37°C et 5% de $CO_2$, les surnageants de culture ont été éliminés, les cellules ont été rincées avec une solution de PBS et les tapis cellulaires mis en Tri-Reagent (sigma T9424) puis immédiatement congelées à -80°C.

L'expression des marqueurs sélectionnés a été évaluée par RT-Q-PCR sur les ARN messagers extraits des cellules correspondant à chaque traitement.

Le marqueur βactine (M12481) a été utilisé comme marqueur de référence.

Les étapes opératoires sont les suivantes:

- Extraction des ARN totaux à l'aide de Tri-Reagent selon le protocole préconisé par le fournisseur. Puis nouvelle extraction par du chloroforme et précipitation à l'isopropanol.

- Elimination des traces d'ADN potentiellement contaminant par traitement avec le système DNA-free (Ambion). Après contrôle de la qualité des ARNs obtenus les trois échantillons correspondant à chaque traitement ont été poolés.
- Réalisation de la réaction de réverse-transcription de l'ARNm en présence de l'amorce oligo(dT) et de l'enzyme Superscript II (Gibco).
- Quantification, par fluorescence, de l'ADNc synthétisé et ajustement des concentrations à 50 ng/μl. Une nouvelle quantification de chaque ADNc, après dilution finale, est réalisée avant la réaction de PCR.

**. PCR Quantitative**

Les réactions de PCR (polymerase chain reactions) ont été réalisées par PCR quantitative avec le système « Light Cycler » (Roche Molecular Systems Inc.) et selon les procédures recommandées par le fournisseur.

**. Analyse des Q-PCR**

L'incorporation de fluorescence dans l'ADN amplifié est menée en continu au cours des cycles de PCR. Ce système permet d'obtenir des courbes de mesure de la fluorescence en fonction des cycles de PCR et d'évaluer ainsi une valeur d'expression relative pour chaque marqueur.

Le nombre de cycles est déterminé à partir des points de « sortie » des courbes de fluorescence. Pour un même marqueur analysé, plus un échantillon sort tard (nombre de cycle élevé), plus le nombre initial de copies de l'ARNm est faible

La valeur de RE (relative expression) est exprimée en unités arbitraires selon la formule suivante :

$$(1/2^{\text{nombre de cycles}}) \times 10^6$$

Résultats: Effets des différents traitements sur l'expression relative du messager **Aquaporine adipeuse (AQPap).** Résultats rapportés à la quantité de messager actine (gène de référence).
RE*= Expression Relative exprimée en unités arbitraires (UA), n=2.

| **AQP7/Actine** | | | |
|---|---|---|---|
| **Traitement** | **Concentration** | **RE*AQP7 (UA)** | **% témoin** |
| ***Contrôle non différencié*** | - | *0.0011* | ***3*** |
| **Témoin différencié** | - | 0.048 | **100** |
| **Escine** | 0.0032 mg/ml<br>0.00064 mg/ml<br>0.000128 mg/ml | 0.043<br>0.039<br>0.034 | **164**<br>**168**<br>**134** |
| **PA2Affilène** | 0.1 mg/ml<br>0.02 mg/ml<br>0.004 mg/ml | 0.026<br>0.051<br>0.051 | **233**<br>**201**<br>**145** |
| **Lycopène encapsulé** | 0.2 %<br>0.04 %<br>0.008 % | 0.025<br>0.024<br>0.022 | **147**<br>**177**<br>**139** |
| (tridec-2-ynylthio)acetic acid | 0.008 mg/ml<br>0.0016 mg/ml<br>0.00032 mg/ml | 0.032<br>0.872<br>0.912 | **174**<br>**105**<br>**130** |
| Fermented sage coumpound (FSC) | 0.04 mg/ml<br>0.008 mg/ml<br>0.0016 mg/ml | 0.020<br>0.020<br>0.025 | **175**<br>**155**<br>**159** |
| {[4-(octylthio)but-2-ynyl]thio}acetic acid | 0.004 mg/ml<br>0.0008 mg/ml<br>0.00016 mg/ml | 0.020<br>0.022<br>0.023 | **167**<br>**153**<br>**128** |

Les résultats sont résumés sur le tableau suivant:

| Nom | Stimulation de mARN |
|---|---|
| Dioschol®<br>0.0006-0.016% | Non |
| Lycopène encapsulé<br>0.008-0.2% | **OUI** |
| Caophénol<br>0.08-2% | Non |
| Escine (α2 bloq)<br>0.128-3.2 μg/ml | **OUI** |
| PA2 Affilene<br>0.004-0.1 mg/ml | **OUI** |
| Fermented sage coumpound (FSC)<br>1.6-40.0μg/ml | **OUI** |
| (tridec-2-ynylthio)acetic acid<br>0.16-4.0μg/ml | **OUI** |
| {[4-(octylthio)but-2-ynyl]thio}acetic acid<br>0.32-8.0μg/ml | **OUI** |
| Lierre<br>0.016-0.4% | Non |

Exemple 3: Composition à usage topique Une émulsion eau/huile est préparée avec la formule suivante:

| Phase aqueuse | |
|---|---|
| Eau | Qs 100 |
| Escine | 0,45 |
| Sulfate de Mg | 0,7 |
| Glycérine | 12 |
| Butylène glycol | 9 |
| Dioschol(1) | 3 |
| Eau Thermale | 5 |
| Éthanol | 20 |
| Phase huileuse | |
| Cyclopentasiloxane | 10 |
| Huile d'abricot | 10 |
| Parfum | 0,3 |

(suite)

| Phase huileuse | |
|---|---|
| DC2-5225C(2) | 10 |

(1) Dioschol : Extrait de racines de Dioscorea opposita (igname sauvage) dans un mélange : dérivé de polyéthylèneglycol (60E) et de mélange de mono-, di-, et triglycéride d'acides caprylique et caprique / conservateurs / glycérine (rapport pondéral 1/93,8/0,2/5) commercialisé par la société Sederma.
(2) DC2 5225 C : mélange de polydiméthylsiloxane oxyéthyléné oxypropyléné (18OE/18OP), de cyclopentasiloxane et d'eau (rapport pondéral 10/88/2) commercialisé par la société Dow Corning,

Exemple 4: composition amincissante à usage topique

| Phase aqueuse | |
|---|---|
| Eau | Qs 100 |
| Caféine | 3 |
| Escine | 0,2 |
| Acide salicylique | 0,72 |
| Sulfate de Mg | 0,7 |
| Citrate trisodique | 2 |
| Glycérine | 8 |
| Butylène glycol | 5 |
| Dioschol(1) | 3 |
| Eau Thermale | 5 |
| Éthanol | 20 |
| Conservateurs | 0,5 |
| Colorants | 0,0001 |
| Neutralisant | 0,72 |
| Phase huileuse | |
| Cyclopentasiloxane | 9 |
| Isoparaffine | 2 |
| Cyclohexasiloxane | 5 |
| Parfum | 0.3 |

(suite)

| Phase huileuse | |
|---|---|
| DC2-5225C(2) | 8 |

(1) Dioschol : Extrait de racines de Dioscorea opposita (igname sauvage) dans un mélange : dérivé de polyéthylèneglycol (6OE) et de mélange de mono-, di-, et triglycéride d'acides caprylique et caprique / conservateurs / glycérine (rapport pondéral 1/93,8/0,2/5) commercialisé par la société Sederma.
(2) DC2 5225 C : mélange de polydiméthylsiloxane oxyéthyléné oxypropyléné (18OE/18OP), de cyclopentasiloxane et d'eau (rapport pondéral 10/88/2) commercialisé par la société Dow Corning,

Les compositions des exemples 3 et 4 sont préparées de manière classique pour l'homme du métier :
La phase aqueuse et la phase huileuse sont préparées séparément à froid. La phase aqueuse est ensuite dispersée sous agitation vigoureuse dans la phase huileuse.

## Revendications

1. Utilisation d'au moins un modulateur de l'aquaglycéroporine adipeuse (AQPap) dans une composition cosmétique en tant qu'agent amincissant pour diminuer le volume des adipocytes, ledit modulateur étant un agent stimulant l'activité AQPap des adipocytes, **caractérisé en ce que** l'on utilise une association (i) d'au moins un modulateur augmentant l'activité intrinsèque de l'AQPap choisi parmi les extraits de poudre de cacao, les sapogénines de Dioscorea opposita et le lycopène encapsulé sous forme de nanocapsules et (ii) d'au moins un modulateur augmentant la synthèse d'AQPap choisi parmi l'escine, les complexes de phospholipides et de proanthocyanidines d'écorce de marron d'Inde et les rétinoïdes.

2. Utilisation d'au moins un modulateur de l'AQPap selon la revendication 1, **caractérisée en ce qu'**au moins modulateur augmente la sortie de glycérol des adipocytes.

3. Utilisation d'au moins un modulateur de l'AQPap selon l'une des revendications 1 ou 2, **caractérisée en ce que** le modulateur stimule l'activité intrinsèque des canaux AQPap en favorisant l'ouverture des canaux AQPap et/ou en favorisant leur translocation vers la membrane.

4. Utilisation d'au moins un modulateur d'AQPap selon au moins l'une des revendications 1 à 3, **caractérisée en ce que** ledit modulateur, quand il est introduit dans une culture d'adipocytes matures, induit une augmentation de la synthèse d'AQPap par ces adipocytes supérieure ou égale à 1,5 fois, par rapport à une culture d'adipocytes matures en absence de modulateur d'AQPap.

5. Utilisation d'un modulateur d'AQPap selon au moins l'une des revendications 1 à 4, **caractérisée en ce que** ledit modulateur augmente d'au moins 1,2 fois la quantité de glycérol libéré dans le surnageant de culture d'adipocytes matures, par rapport à la quantité de glycérol dans le surnageant de culture d'adipocytes matures n'ayant pas reçu de modulateur d'AQPap.

6. Utilisation d'un modulateur d'AQPap selon au moins l'une des revendications 1 à 5, **caractérisée en ce que** la composition contient en outre au moins un agent amincissant lipolytique et/ou inhibiteur de la lipogénèse et/ou inhibiteur de la différenciation adipocytaire.

7. Utilisation d'un modulateur d'AQPap selon la revendication 6, **caractérisée en ce que** l'actif amincissant lipolytique est choisi dans le groupe comprenant les inhibiteurs de phosphodiestérase, les inhibiteurs des récepteurs $\alpha$-2, les inhibiteurs des récepteurs NPY, les inhibiteurs de la synthèse des récepteurs aux LDL ou VLDL, les activateurs des récepteurs $\beta$, les activateurs des protéines G, les bloqueurs du transport du glucose, les activateurs de l'adénylcyclase, les peptides ou protéines lipolytiques.

8. Utilisation d'au moins un modulateur d'AQPap selon au moins l'une des revendications 1 à 7, **caractérisée en ce que** les dits modulateurs sont présents dans la composition à une concentration de 0,001 à 20% en poids par rapport au poids total de la composition.

9. Utilisation selon au moins l'une des revendications 1 à 8, **caractérisée en ce que** la composition est une composition à usage topique.

10. Utilisation selon au moins l'une des revendications 1 à 9, **caractérisée en ce que** la composition contient en outre au moins un agent choisi parmi les agents desquamants, les agents hydratants, les agents raffermissants, les modulateurs d'aquaporines, les agents promoteurs de synthèse des constituants de la matrice extra-cellulaire ou inhibiteurs de leur dégradation, les agents actifs sur la microcirculation, les agents actifs sur la métabolisme énergétique des cellules.

11. Utilisation selon au moins l'une des revendications 1 à 10, **caractérisée en ce que** la composition est sous la sous la forme de solution aqueuse, alcoolique, hydro-alcoolique ou huileuse, de suspension, de dispersion, d'émulsions E/H, H/E ou multiple, de gels aqueux ou anhydres, de dispersion vésiculaires de type ionique ou non ionique.

12. Utilisation selon au moins l'une des revendications 1 à 8, **caractérisée en ce que** la composition est une composition pour la voie orale.

13. Utilisation selon au moins l'une des revendications 1 à 8 ou 12, **caractérisée en ce que** la composition contient en outre au moins un composé choisi parmi les sels de Calcium, des modulateurs de canaux calciques , l'acide conjugué linoléique, les modulateurs de la production de NO, les modulateurs directs ou indirects de la nitric oxide synthase, la leucine ou les acides aminés branchés.

14. Composition cosmétique **caractérisé en ce qu'**elle contient une association d'au moins (i) un agent modulateur augmentant l'activité intrinsèque de l'AQPap choisi parmi les extraits de poudre de cacao, les sapogénines de Dioscorea opposita et le lycopène encapsulé sous forme de nanocapsules et (ii) un agent modulateur augmentant la synthèse d'AQPap choisi parmi l'escine, les complexes de phospholipides et de proanthocyanidines d'écorce de marron d'Inde, et les rétinoïdes, et (iii) d'au moins un actif lipolytique.

## Claims

1. Use of at least one modulator of aquaglyceroporin adipose (AQPap) in a cosmetic composition as slimming agent for reducing the volume of adipocytes, the said modulator being an agent which stimulates the AQPap activity of the adipocytes, **characterized in that** use is made of a combination (i) of at least one modulator which increases the intrinsic activity of the AQPap chosen from cocoa powder extracts, Dioscorea opposita sapogenins and lycopene encapsulated in the form of nanocapsules and (ii) of at least one modulator which enhances the synthesis of AQPap chosen from aescin, complexes of phospholipids and of horse chestnut bark proanthocyanidins, and retinoids.

2. Use of at least one AQPap modulator according to Claim 1, **characterized in that** the modulator enhances the departure of glycerol from the adipocytes.

3. Use of at least one AQPap modulator according to either of Claims 1 and 2, **characterized in that** the modulator stimulates the intrinsic activity of the AQPap channels by promoting the opening of the AQPap channels and/or by promoting their translocation towards the membrane.

4. Use of at least one AQPap modulator according to at least one of Claims 1 to 3, **characterized in that** the said modulator, when it is introduced into a culture of mature adipocytes, induces an increase in the synthesis of AQPap by these adipocytes of greater than or equal to 1.5 times, with respect to a culture of mature adipocytes in the absence of AQPap modulator.

5. Use of an AQPap modulator according to at least one of Claims 1 to 4, **characterized in that** the said modulator increases by at least 1.2 times the amount of glycerol released into the culture supernatant of mature adipocytes, with respect to the amount of glycerol in the culture supernatant of mature adipocytes which have not received AQPap modulator.

6. Use of an AQPap modulator according to at least one of Claims 1 to 5, **characterized in that** the composition additionally comprises at least one lipolytic slimming agent and/or lipogenesis inhibitor and/or adipocyte differentiation inhibitor.

7. Use of an AQPap modulator according to Claim 6, **characterized in that** the lipolytic slimming active agent is chosen from the group consisting of phosphodiesterase inhibitors, inhibitors of $\alpha_2$ receptors, inhibitors of NPY receptors, inhibitors of the synthesis of LDL or VLDL receptors, activators of $\beta$ receptors, activators of G-proteins, glucose transportation blockers, adenylate cyclase activators, lipolytic peptides and lipolytic proteins.

8. Use of at least one AQPap modulator according to at least one of Claims 1 to 7, **characterized in that** the said modulators are present in the composition at a concentration of 0.001 to 20% by weight with respect to the total weight of the composition.

9. Use according to at least one of Claims 1 to 8, **characterized in that** the composition is a composition for topical use.

10. Use according to at least one of Claims 1 to 9, **characterized in that** the composition additionally comprises at least one agent chosen from desquamating agents, moisturizing agents, firming agents, aquaporin modulators, agents which promote the synthesis of the constituents of the extracellular matrix or which inhibit their decomposition, agents active with regard to microcirculation, or agents active with regard to the energy metabolism of the cells.

11. Use according to at least one of Claims 1 to 10, **characterized in that** the composition is in the form of an aqueous, alcoholic, aqueous/alcoholic or oily solution, of a suspension, of a dispersion, of W/O, O/W or multiple emulsions, of aqueous or anhydrous gels or of vesicular dispersions of ionic or nonionic type.

12. Use according to at least one of Claims 1 to 8, **characterized in that** the composition is a composition for the oral route.

13. Use according to at least one of Claims 1 to 8 or 12, **characterized in that** the composition additionally comprises at least one compound chosen from calcium salts, calcium channel modulators, conjugated linoleic acid, NO production modulators, direct or indirect modulators of nitric oxide synthase, leucine or branched amino acids.

14. Cosmetic composition, **characterized in that** it comprises a combination of at least (i) one modulating agent which increases the intrinsic activity of the AQPap chosen from cocoa powder extracts, Dioscorea opposita sapogenins and lycopene encapsulated in the form of nanocapsules and (ii) one modulating agent which enhances the synthesis of AQPap chosen from aescin, complexes of phospholipids and of horse chestnut bark proanthocyanidins, and retinoids, and (iii) of at least one lipolytic active agent.

**Patentansprüche**

1. Verwendung mindestens eines Modulators von Aquaglyceroporin des Fettgewebes (AQPap) in einer kosmetischen Zusammensetzung als schlanker machendes Mittel zur Verminderung des Volumens von Adipocyten, wobei der Modulator ein Stoff ist, der die AQPap-Aktivität der Adipocyten stimuliert, **dadurch gekennzeichnet, dass** eine Kombination (i) mindestens eines Modulators, der die intrinsische Aktivität von AQPap erhöht und unter den Extrakten von Kakaopulver, Sapogeninen von Dioscorea opposita und in Form von Nanokapseln eingekapseltem Lycopin ausgewählt ist, und (ii) mindestens eines Modulators verwendet wird, der die Synthese von AQPap fördert und unter Aescin, Komplexen von Phospholipiden und Proanthocyanidinen der Rosskastanienrinde und Retinoiden ausgewählt ist.

2. Verwendung mindestens eines Modulators von AQPap nach Anspruch 1, **dadurch gekennzeichnet, dass** der mindestens eine Modulator den Glycerol-Abtransport der Adipocyten verbessert.

3. Verwendung mindestens eines Modulators von AQPap nach einem der Ansprüche 1 oder 2, **dadurch gekennzeichnet, dass** der Modulator die intrinsische Aktivität der AQPap-Kanäle stimuliert, indem er das Öffnen der

AQPap-Kanäle fördert und/oder ihre Translokation in die Membran begünstigt.

4. Verwendung mindestens eines AQPap-Modulators nach einem der Ansprüche 1 bis 3, **dadurch gekennzeichnet, dass** der Modulator, wenn er in eine Kultur reifer Adipocyten eingebracht wird, im Vergleich mit einer Kultur reifer Adipocyten bei Abwesenheit des AQPap-Modulators eine Steigerung der Synthese von AQPap durch die Adipocyten größer oder gleich des 1,5-Fachen induziert.

5. Verwendung eines AQPap-Modulators nach einem der Ansprüche 1 bis 4, **dadurch gekennzeichnet, dass** der Modulator die Menge des in dem Überstand der Kultur von reifen Adipocyten freigesetzten Glycerols im Vergleich mit der Menge des Glycerols in dem Überstand der Kultur reifer Adipocyten, in die kein AQPap-Modulator gegeben wurde, um mindestens das 1,2-Fache erhöht.

6. Verwendung eines AQPap-Modulators nach einem der Ansprüche 1 bis 5, **dadurch gekennzeichnet, dass** die Zusammensetzung ferner mindestens einen schlanker machenden lipolytischen Stoff und/oder Inhibitor der Lipogenese und/oder Inhibitor der Differenzierung der Adipocyten enthält.

7. Verwendung eines AQPap-Modulators nach Anspruch 6, **dadurch gekennzeichnet, dass** der schlanker machende lipolytische Wirkstoff unter den Inhibitoren der Phosphodiesterase, Inhibitoren der $\alpha$2-Rezeptoren, Inhibitoren von NPY-Rezeptoren, Inhibitoren der Synthese von LDL- oder VLDL-Rezeptoren, Aktivatoren von $\beta$-Rezeptoren, Aktivatoren von G-Proteinen, Blockern des Glucosetransports, Aktivatoren der Adenylcyclase, lipolytischen Peptiden oder Proteinen ausgewählt ist.

8. Verwendung eines AQPap-Modulators nach einem der Ansprüche 1 bis 7, **dadurch gekennzeichnet, dass** die Modulatoren in der Zusammensetzung in einer Konzentration von 0,001 bis 20 Gew.-%, bezogen auf das Gesamtgewicht der Zusammensetzung, enthalten sind.

9. Verwendung nach mindestens einem der Ansprüche 1 bis 8, **dadurch gekennzeichnet, dass** die Zusammensetzung eine Zusammensetzung für die topische Anwendung ist.

10. Verwendung nach mindestens einem der Ansprüche 1 bis 9, **dadurch gekennzeichnet, dass** die Zusammensetzung ferner mindestens einen Stoff enthält, der unter den abschuppenden Wirkstoffen, Hydratisierungsmitteln, straffenden Wirkstoffen, Modulatoren der Aquaporine, Wirkstoffen, die die Synthese der Bestandteile der extracellulären Matrix fördern oder deren Zersetzung inhibieren, Stoffen, die auf die Mikrozirkulation einwirken, Stoffen, die auf den Energiehaushalt der Zellen einwirken, ausgewählt ist.

11. Verwendung nach mindestens einem der Ansprüche 1 bis 10, **dadurch gekennzeichnet, dass** die Zusammensetzung als wässrige, alkoholische, wässrig-alkoholische oder ölige Lösung, Suspension, Dispersion, W/O-Emulsion, O/W-Emulsion oder multiple Emulsion, als wässriges oder wasserfreies Gel, als Vesikeldispersion vom ionischen oder nichtionischen Typ vorliegt.

12. Verwendung nach mindestens einem der Ansprüche 1 bis 8, **dadurch gekennzeichnet, dass** die Zusammensetzung eine Zusammensetzung für die orale Verabreichung ist.

13. Verwendung nach mindestens einer Ansprüche 1 bis 8 oder 12, **dadurch gekennzeichnet, dass** die Zusammensetzung ferner mindestens eine Verbindung enthält, die unter den Calciumsalzen, Modulatoren der Calciumkanäle, der konjugierten Linolsäure, Modulatoren der NO-Bildung, direkten oder indirekten Modulatoren der NO-Synthase, Leucin und verzweigten Aminosäuen ausgewählt ist.

14. Kosmetische Zusammensetzung, **dadurch gekennzeichnet, dass** sie die Kombination (i) mindestens eines Modulators, der die intrinsische Aktivität von AQPap erhöht und unter den Extrakten von Kakaopulver, Sapogeninen von Dioscorea opposita und in Form von Nanokapseln eingekapseltem Lycopin ausgewählt ist, und (ii) mindestens eines Modulators, der die Synthese von AQPap fördert und unter Aescin, Komplexen von Phospholipiden und Proanthocyanidinen der Rosskastanienrinde und Retinoiden ausgewählt ist, und (iii) mindestens einen lipolytischen Wirkstoff enthält.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2617401 A
- EP 371844 A
- FR 2669537 A
- US 5194259 A
- WO 0030603 A
- FR 2819409
- US 6399089 B
- WO 0164177 A
- FR 2578165
- FR 2774589
- WO 02089758 A
- FR 2790645
- WO 9215293 A
- US 20020106388 A
- FR 2831058
- WO 0137799 A
- EP 1084700 A
- FR 2788058
- FR 2781231
- FR 2786693
- FR 2758724
- FR 2782921
- FR 2774292
- EP 0852949 A
- WO 0164219 A
- WO 0112225 A
- EP 1345919 A
- EP 1292608 A


### Littérature non-brevet citée dans la description


- **P. VALET.** *J. Clin. Invest.,* 1990, vol. 85, 291-295
- *Biochem. Biophys. Res. Commun.,* 1997, vol. 241, 53-58
- **KISHIDA et al.** *J.Biol.Chem.,* 2001, vol. 276, 36251-36260
- **P. AGRE et al.** *J. of Physiology,* 2002, 542.1